# EUROPEAN PATENT APPLICATION

(11) **EP 4 589 595 A1**
(43) Date of publication of application: **23.07.2025**
(21) Application number: 22963991.9
(22) Date of filing: 04.11.2022
(51) Int. Cl.: G16B 20/50

(54) **METHOD AND APPARATUS FOR DETERMINING LOSS-OF-FUNCTION EVIDENCE OF PATHOGENICITY**

(71) Applicant: BGI Genomics Co., Limited, Guangdong 518083 (CN)
(72) Inventor: LI, Hong, Shenzhen, Guangdong 518083 (CN); ZHANG, Yu, Shenzhen, Guangdong 518083 (CN); XIA, Menglei, Shenzhen, Guangdong 518083 (CN); WEI, Yi, Shenzhen, Guangdong 518083 (CN); XIONG, Yun, Shenzhen, Guangdong 518083 (CN); YUAN, Yuying, Shenzhen, Guangdong 518083 (CN)
(74) Representative: Potter Clarkson
(86) International application number: PCT/CN2022/129694
(87) International publication number: WO 2024/092681

(57) **Abstract**

A method and apparatus for determining loss-of-function evidence of pathogenicity. The method comprises: according to the effect of a target variant on transcription, obtaining the category of the target variant; based on this category of the target variant, determining the effect of the target variant on a target gene function; and according to the result obtained of the effect of the target variant on the target gene function, determining whether target variant information is loss-of-function evidence of pathogenicity of the target variant. This classification enables a swift evaluation of the variant's impact on gene function. Furthermore, according to the result of the effect of the target variant on the target gene function, it can be determined whether the target variant information is loss-of-function evidence of pathogenicity of the target variant, thereby determining the loss-of-function evidence of pathogenicity.

## Description

### FIELD

The present application relates to the field of bioinformatics, particularly a method and apparatus for determining loss-of-function pathogenicity evidence.

### BACKGROUND

With the development and popularization of genetic technology, various laboratories have detected more and more new sequence variations. Interpreting the clinical significance of a given sequence variation and clarifying the relationship between the variant sequence and the diseases are the key points to achieve precision medicine for diseases.

Currently, manual interpretation is usually based on the guidelines issued by the American College of Medical Genetics and Genomics (ACMG) in 2015 (Richards S, Aziz N, Bale S, et al. Standards and guidelines for the interpretation of sequence variants: a joint consensus recommendation of the American College of Medical Genetics and Genomics and the Association for Molecular Pathology[J]. Genetics in medicine, 2015, 17(5): 405-423.). In the process of interpretation, it is necessary to use a variety of professional databases and prediction software and look up a large amount of medical literature for evidence-based interpretation. At the same time, interpreters are required to have a multidisciplinary background in molecular biology, genetics, medicine, etc.

A study showed that the pathogenicity classification results of 99 variant sequences from nine top laboratories in the United States were evaluated under the premise of using the aforementioned ACMG guidelines. The consistency of the interpretation results was only 34%. After multi-party consultation and detailed review of the ACMG standards, the consistency of the interpretation results increased to 71%. (Amendola L M, Jarvik G P, Leo M C, et al. Performance of ACMG-AMP variant-interpretation guidelines among nine laboratories in the Clinical Sequencing Exploratory Research Consortium[J]. The American Journal of Human Genetics, 2016, 98(6): 1067-1076.) It can be seen that the consistency of the interpretation results cannot be guaranteed based on the personal understanding and experience accumulation of the interpreters.

Among the 28 variant grading criteria specified in the ACMG guidelines, there is only one very strong pathogenicity evidence, which is crucial to determine the pathogenicity of variations that cause loss of gene function (LOF). Specifically, "when the pathogenic mechanism of a disease is loss of function (LOF), there are no functional variations (nonsense mutations, frameshift mutations, classic ±1 or 2 splicing mutations, initiation codon mutations, single or multiple exon deletions)", i.e., loss-of-function pathogenicity evidence. According to the combined standard rules for the classification of genetic variant specified in the guidelines, the one very strong pathogenicity evidence only needs to be combined with 1-2 other levels of pathogenicity evidence to determine that the variant is likely pathogenic or pathogenic. According to statistics, the average usage rate of loss-of-function pathogenicity evidence is about 20%, ranking fourth among all evidence. In actual use, there are still cases of artificial adjustment of the strength of the evidence. However, the detailed usage rules of this evidence are not perfect.

The determination of the loss-of-function pathogenicity evidence usually relies on manual work, which requires very complicated determination processes, involving the pathogenic mechanisms, biologically relevant transcripts, nonsense-mediated mRNA decay (NMD) prediction, gene function prediction and other aspects. At the same time, different types of nonfunctional variations have different biological characteristics, and their determination methods are also different. Among the various types of nonfunctional variations, the effects of nonsense mutations, frameshift mutations, classic ±1 or 2 splicing mutations, and initiation codon mutations on the gene function are relatively easy to predict. In particular, for the nonfunctional variations involving single or multiple exons, the specific location of the variation, the variation length and other aspects are involved, and the situation is more complicated. Conventional variant detection or annotation tools lack the prediction of the influences of the variations on the encoded protein. As a result of the above reasons, in actual operation, the guidelines are usually not followed for determination, or the determination is made only based on personal experience, or it is directly ignored. Existing interpretations mostly rely on manual work and personal understanding of the interpretation guidelines. However, the interpretation guidelines only provide principled guidance and are thus not very operational. Thus, the standards are not unified, and the consistency, accuracy and efficiency are low.

### SUMMARY

An embodiment provides a method for determining loss-of-function pathogenicity evidence. The method includes: Step S101 of obtaining, based on an influence of a target variation on transcription, a category of the target variation; Step S102 of determining, based on the category of the target variation, an influence of the target variation on functions of a target gene, where the target gene is a gene where the target variation occurs; and Step S103 of determining, based on a consequence of the influence obtained in the Step S102 of the target variation on the functions of the target gene, whether target variation information is loss-of-function pathogenicity evidence of the target variation

The target variation information is the category of the target variation and/or the consequence of the influence in the target variation information.

In the method for determining the loss-of-function pathogenicity evidence provided by the present application, the influence of the target variation on the functions of the target gene can be quickly determined based on the category of the target variation. Further, based on the consequence of the influence of the target variation on the functions of the target gene, the loss-of-function pathogenicity evidence can be determined by determining whether the target variation information meets the requirement for the loss-of-function pathogenicity evidence of the target variation.

### BRIEF DESCRIPTION OF THE DRAWINGS

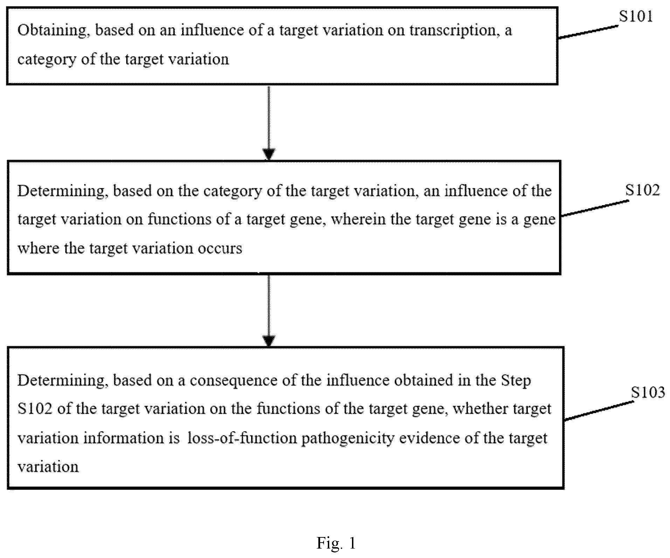

Fig. 1 is a flow chart of a method for determining loss-of-function pathogenicity evidence provided by an embodiment of the present application;

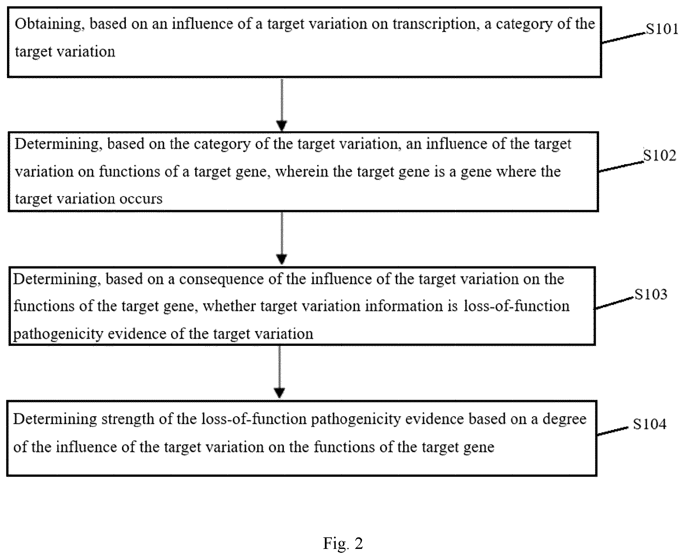

Fig. 2 is a flow chart of a method for determining strength of loss-of-function pathogenicity evidence provided by an embodiment of the present application; and

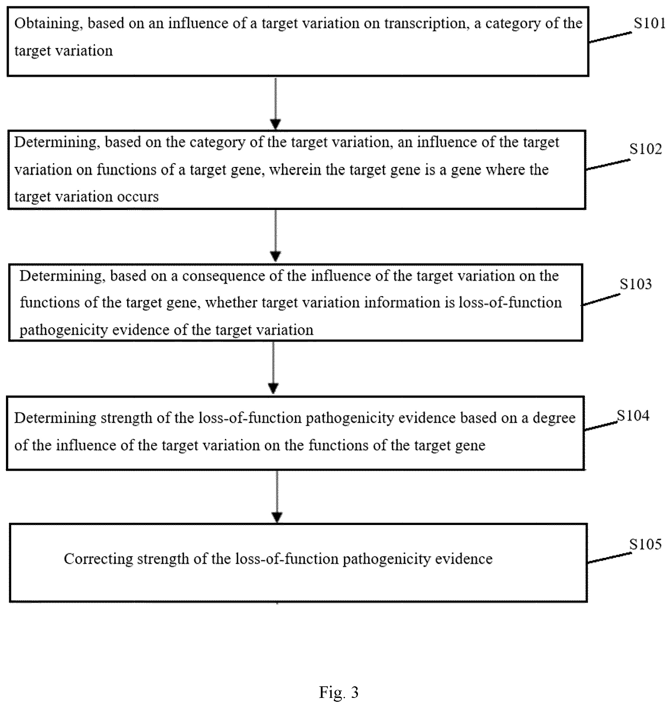

Fig. 3 is a flow chart of a method for correcting strength of loss-of-function pathogenicity evidence provided by an embodiment of the present application.

### DETAILED DESCRIPTION

The present application is further described in detail below by specific embodiments. In the following embodiments, many details are described to make the present application better understood. However, those skilled in the art can recognize without difficulty that some characteristics can be omitted in different situations, or can be replaced by other materials and methods. In some cases, some operations related to the present application are not shown or described in the specification, in order to avoid the core part of the present application from being overwhelmed by too much description, and those skilled in the art, even without describing the related operations in detail, can fully understand the related operations according to the description in the specification and the general technical knowledge in the art.

In addition, the features, operations or characteristics described in the specification can be combined in any appropriate manner to form various implementations. At the same time, the steps or actions in the method description can also be interchanged or adjusted in a manner that is obvious to those skilled in the art. Therefore, the various sequences in the specification are only for the purpose of clearly describing a certain embodiment and are not meant to be a required sequence, unless otherwise specified that a certain sequence must be followed.

The serial numbers assigned to the components herein, such as "first" and "second", are only used to distinguish the objects described and do not have any order or technical meaning.

As used herein, "coding sequence (CDS)" refers to a coding sequence region in mature mRNA that can be translated into amino acids, starting from an initiation codon and ending with a termination codon.

As used herein, "5' untranslated region" (abbreviated as 5'UTR) is a short sequence between a cap and the initiation codon of the coding region, including a sequence that marks the translation initiation. 5'UTR is a highly sensitive region for translation initiation, and its length, secondary structure, and the number of AUGs will affect the efficiency of translation initiation.

As used herein, "open reading frame (ORF)" is a normal nucleotide sequence of a structural gene, and a complete polypeptide chain is encoded by the reading frame from the initiation codon to the termination codon, between which no termination codon interrupting the translation exists. In the mRNA sequence, every three consecutive bases (i.e., a triplet "codon") encode the corresponding amino acid.

Herein, "judge", "determination" and "determine" are used interchangeably.

Herein, the "target variation" may include one or more variation categories (two or more variation categories). The method for detecting the target variation is not limited, for example, the first-generation sequencing, the second-generation sequencing, the third-generation sequencing, and PCR. The diseases associated with the target variation are not limited, for example, various single-gene genetic diseases, and various hereditary tumors.

Herein, "influence" and "damage" are synonymous. When the target variation causes a change of the functions of the target gene, it is determined that the target variation has an influence on the functions of the target gene, causing damages to the functions of the target gene. The determination of the influence of the target variation on the functions of the target gene specifically refers to determining whether the target variation damages the functions of the target gene. The target variation having an influence on the functions of the target gene specifically refers to the target variation damaging the functions of the target gene. The target variation having unclear influence on the functions of the target gene specifically refers to the situation where it cannot be determined that the functions of the target gene is damaged.

In an embodiment, the method for determining the influence of a variation on a gene function provided by the present application is applicable to nonfunctional variations, such as nonsense mutations, frameshift mutations, classic ± 1 or 2 splicing mutations, and single- or multiple-exon deletion variation. The method provided by the present application is particularly applicable to single- or multiple-exon deletion variation. Compared with the prior art, the present application provides an executable technical solution with more prominent technical effects.

In an embodiment, the target variation may be of one or more categories. In addition to a first category and a second category, the target variation may also include other categories, such as variations that cause change in ORF and NMD.

In an embodiment, the source of information on the last pathogenic variation at the 3' end of the target gene and the important functional domain of the target gene is not limited, and can be derived from literature reports or recorded in the public database. For example, the former may come from https://www.ncbi.nlm.nih.gov/clinvar/, and the latter may come from https://asia.ensembl.org/index.html. In addition, the collected information on the last pathogenic variation at the 3' end of the target gene and the important functional domain of the target gene can be generated into a datasheet or a custom configuration library, respectively, for direct call.

Herein, the "region affected by the target variation" refers to all the regions that differ from the reference sequence due to the target variation.

Herein, the " proportion of the region affected by the target variation" refers to a ratio of a length of the regions affected by the target variation to a length of the reference sequence.

Herein, the "initiation codon pathogenicity of the target gene" means that other variations (non-target variations) at the initiation codon of the target gene are determined as pathogenic based on industry standards and/or expert consensus, for example, according to the pathogenic (P) or likely pathogenic (LP) results given according to the guidelines issued by the American College of Medical Genetics and Genomics (ACMG). The source of information on the initiation codon pathogenicity reports of the target gene is not limited, which can derive from the literature reports or the records in the public database (for example, https://www.ncbi.nlm.nih.gov/clinvar/). In addition, the collected information on the initiation codon pathogenicity reports of the target gene can be generated into a datasheet or a custom configuration library for direct call.

According to a first aspect, an embodiment, a method for determining loss-of-function pathogenicity evidence is provided. The method includes: step S101 of obtaining, based on an influence of a target variation on transcription, a category of the target variation; step S102 of determining, based on the category of the target variation, an influence of the target variation on functions of a target gene, wherein the target gene is a gene where the target variation occurs; and step S103 of determining, based on a consequence of the influence obtained in the step S102 of the target variation on the functions of the target gene, whether target variation information is loss-of-function pathogenicity evidence of the target variation. The target variation information is the category of the target variation and/or the consequence of the influence in the target variation information.

It is crucial in the process of determining the loss-of-function pathogenicity evidence to determine the influence of the target variation on the functions of the target gene. By obtaining the category of the target variation, the influence of the target variation on the functions of the target gene can be quickly determined. According to the influence of the target variation on the functions of the target gene, it can be determined whether the requirements for the loss-of-function pathogenicity evidence is met, thereby determining that the target variation information is the loss-of-function pathogenicity evidence of the target variation.

In an embodiment, the target variation is a single- or multiple-exon deletion variation.

In an embodiment, the influence of the target variation on transcription includes: whether the target variation causes a change in an Open Reading Frame (ORF) and/or whether the target variation causes Nonsense-Mediated mRNA Decay (NMD).

In an embodiment, the category of the target variation includes one or more of a first category, a second category, a third category, and a fourth category. The first category includes variations that cause a change in ORF and do not cause the NMD. The second category includes variations that do not cause a change in ORF. The third category includes variations that cover the first CDS but are not whole gene deletion. The fourth category includes variations that cover the last CDS but are not the whole gene deletion.

In an embodiment, if the target variation has an influence on the functions of the target gene, the target variation information is determined as the loss-of-function pathogenicity evidence of the target variation; and if the target variation has no influence on the functions of the target gene, the target variation information is determined to be not the loss-of-function pathogenicity evidence of the target variation.

In the present embodiment, as an example, the category of the target variation is the first category. When the category of the target variation is the first category, it can be directly determined that the target variation has an influence on the functions of the target gene, thereby determining that the first category is the loss-of-function pathogenicity evidence of the target variation, and in this case, the target variation information is the first category.

If it cannot be directly determined based on the category of the target variation whether the target variation has an influence on the functions of the target gene, the influence of the target variation on the gene function is required to be further determined, and the consequence of the influence obtained by the further determination is the loss-of-function pathogenicity evidence of the target variation.

In an embodiment, when the category of the target variation includes the first category, the second category, the third category, or the fourth category, it is determined that the target variation has an influence on the functions of the target gene, and that the category of the target variation is the loss-of-function pathogenicity evidence of the target variation. The first category includes variations that cause a change in ORF and do not cause the NMD. The second category includes variations that do not cause a change in ORF. The third category includes variations that cover the first CDS but are not whole gene deletion. The fourth category includes variations that cover the last CDS but are not the whole gene deletion.

It can be understood that when the target variation is of the first category, the target variation information is the first category, and the first category is determined as the loss-of-function pathogenicity evidence of the target variation; when the target variation is of the second category, the target variation information is the second category, and the second category is determined as the loss-of-function pathogenicity evidence of the target variation; when the target variation is of the third category, the target variation information is the third category, and the third category is determined as the loss-of-function pathogenicity evidence of the target variation; and when the target variation is of the fourth category, the target variation information is the fourth category, and the fourth category is determined as the loss-of-function pathogenicity evidence of the target variation.

In an embodiment, when the category of the target variation includes a fifth category and/or a sixth category, the target variation has no influence on the functions of the target gene, and the category of the target variation is not the loss-of-function pathogenicity evidence of the target variation. The fifth category includes variations that are uncapable of being annotated to coding transcripts. The sixth category includes variations that are capable of being annotated to the coding transcripts and enable a region affected by the target variation to not include a coding sequence.

It can be understood that when the category of the target variation is the fifth category and/or the sixth category, the fifth category and/or the sixth category are/is not the loss-of-function pathogenicity evidence of the target variation. In the process of determining the loss-of-function pathogenicity evidence, the fifth category and/or the sixth category can be directly excluded.

According to a second aspect, in an embodiment, a method for determining strength of loss-of-function pathogenicity evidence. The method includes: step S104 of determining strength of the loss-of-function pathogenicity evidence obtained by the method of the first aspect based on a degree of the influence of the target variation on the functions of the target gene.

In the present embodiment, the degree of the influence of the target variation on the functions of the target gene is positively correlated with the strength of the loss-of-function pathogenicity evidence.

In the present application, for different categories of target variations, different treatment methods are adopted to determine the degree of the influence of the target variation on the functions of the target gene, thereby determining the strength of the loss-of-function pathogenicity evidence.

In an embodiment, the degree of the influence of the target variation on the functions of the target gene is assessed through a first treatment and/or a second treatment.

The first treatment includes: determining the degree of the influence of the target variation on the functions of the target gene by comparing a positional relationship between the target variation and a last pathogenic variation at the 3' end of the target gene.

The second treatment includes: determining the degree of the influence of the target variation on the functions of the target gene by comparing a positional relationship between a region affected by the target variation and an important functional domain of the target gene.

In an embodiment, in the first treatment, if the target variation is located upstream of the last pathogenic variation at the 3' end of the target gene, the target variation information is determined as a strong loss-of-function pathogenicity evidence of the target variation, and the target variation information includes the category of the target variation and the target variation being located upstream of the last pathogenic variation at the 3' end of the target gene; and in the second treatment, if the region affected by the target variation overlaps with the important functional domain of the target gene, the target variation information is determined as a strong loss-of-function pathogenicity evidence of the target variation, and the target variation information includes the category of the target variation and the region affected by the target variation overlapping with the important functional domain of the target gene.

It can be understood that, in the first treatment, if the target variation is located upstream of the last pathogenic variation at the 3' end of the target gene, it can be determined that the target variation has a great influence on the functions of the target gene, and therefore the target variation information is determined as the strong loss-of-function pathogenicity evidence of the target variation.

In this case, the target variation information includes the category of the target variation and the target variation being located upstream of the last pathogenic variation at the 3' end of the target gene. That is, the target variation information is the category of the target variation and the consequence of the influence of the target variation on the functions of the target gene. For example, if the category of the target variation is the first category, the target variation information includes causing the change in the ORF and not causing the NMD, and the target variation being located upstream of the last pathogenic variation at the 3' end of the target gene, and thus the target variation information is determined as a strong loss-of-function pathogenicity evidence of the target variation (or the strength of the loss-of-function pathogenicity evidence of the target variation is: strong).

It can be understood that in the second treatment, if the region affected by the target variation overlaps with the important functional domain of the target gene, it can be determined that the target variation has a great influence on the functions of the target gene, and therefore the target variation information is determined as the strong functional pathogenicity evidence of the target variation.

In this case, the target variation information includes the category of the target variation and the region affected by the target variation overlapping with the important functional domain of the target gene. That is, the target variation information is the category of the target variation and the consequence of the influence of the target variation on the functions of the target gene. For example, if the category of the target variation is the second category, the target variation information includes not causing a change in ORF and the region affected by the target variation overlapping with the important functional domain of the target gene, and thus the target variation information is determined as the strong loss-of-function pathogenicity evidence of the target variation (or the strength of the loss-of-function pathogenicity evidence of the target variation is: strong).

In an embodiment, in the first treatment, if the target variation is located downstream of the last pathogenic variation at the 3' end of the target gene, the degree of the influence of the target variation on the functions of the target gene is determined based on a proportion of the region affected by the target variation.

In the second treatment, if the region affected by the target variation does not overlap with the important functional domain of the target gene, the degree of the influence of the target variation on the functions of the target gene is determined based on a proportion of the region affected by the target variation.

It can be understood that in the first treatment, if the target variation is located downstream of the last pathogenic variation at the 3' end of the target gene, the degree of the influence of the target variation on the target gene is unclear. Thus, the degree of the influence of the target variation on the functions of the target gene is required to be further determined based on the proportion of the region affected by the target variation, to determine the strength of the loss-of-function pathogenicity evidence of the target variation.

In the second treatment, if the region affected by the target variation does not overlap with the important functional domain of the target gene, the degree of the influence of the target variation on the target gene is unclear. Thus, the degree of the influence of the target variation on the functions of the target gene is required to be further determined based on the proportion of the region affected by the target variation, to determine the strength of the loss-of-function pathogenicity evidence of the target variation.

In an embodiment, determining the degree of the influence of the target variation on the functions of the target gene based on the proportion of the region affected by the target variation includes: when the proportion of the region affected by the target variation is greater than or equal to a preset value, determining the target variation information as the strong loss-of-function pathogenicity evidence of the target variation. In the first treatment, the target variation information includes the category of the target variation, the target variation being located downstream of the last pathogenic mutation at the 3' end of the target gene, and the proportion of the region affected by the target variation being greater than or equal to the preset value. In the second treatment, the target variation information includes the category of the target variation, the region affected by the target variation not overlapping with the important functional domain of the target gene, and the proportion of the region affected by the target variation being greater than or equal to the preset value.

In the present embodiment, in the first treatment, if the category of the target variation is the first category, the target variation information includes: causing the change in the open reading frame and not causing the NMD, the target variation being located downstream of the last pathogenic variation at the 3' end of the target gene, and the proportion of the region affected by the target variation being greater than or equal to the preset value. Thus, the target variation information is determined as the strong loss-of-function pathogenicity evidence of the target variation (or the strength of the loss-of-function pathogenicity evidence of the target variation is: strong).

In the second treatment, if the category of the target variation is the second category, the target variation information includes: not causing the change in the open reading frame, the region affected by the target variation not overlapping with the important functional domain of the target gene, and the proportion of the region affected by the target variation being greater than or equal to the preset value. Thus, the target variation information is determined as the strong loss-of-function pathogenicity evidence of the target variation (or the strength of the loss-of-function pathogenicity evidence of the target variation is: strong).

Preferably, the determining the degree of the influence of the target variation on the functions of the target gene based on the proportion of the region affected by the target variation further includes: when the proportion of the region affected by the target variation is smaller than the preset value, determining the target variation information as a moderate loss-of-function pathogenicity evidence of the target variation. In the first treatment, the target variation information includes the category of the target variation, the target variation being located downstream of the last pathogenic variation at the 3' end of the target gene, and the proportion of the region affected by the target variation being smaller than the preset value. In the second treatment, the target variation information includes the category of the target variation, the region affected by the target variation not overlapping with the important functional domain of the target gene, and the proportion of the region affected by the target variation being smaller than the preset value.

In the present embodiment, in the first treatment, if the category of the target variation is the first category, the target variation information includes: causing the change in the open reading frame and not causing the NMD, the target variation being located downstream of the last pathogenic variation at the 3' end of the target gene, and the proportion of the region affected by the target variation being smaller than the preset value. Thus, the target variation information is determined as the moderate loss-of-function pathogenicity evidence of the target variation.

In the second treatment, if the category of the target variation is the second category, the target variation information includes: not causing the change in the open reading frame, the region affected by the target variation not overlapping with the important functional domain of the target gene, and the proportion of the region affected by the target variation being smaller than the preset value. Thus, the target variation information is determined as the moderate loss-of-function pathogenicity evidence of the target variation.

In an embodiment, in the first treatment, the category of the target variation is the first category. In the second treatment, the category of the target variation is the second category. The first category includes variations that cause a change in ORF and do not cause the NMD. The second category includes variations that do not cause a change in ORF.

It can be understood that in the present embodiment, when the category of the target variation is the first category, the first treatment is used for determining the degree of the influence of the target variation on the target gene; and when the category of the target variation is the second category, the second treatment is used for determining the degree of the influence of the target variation on the target gene.

In an embodiment, the degree of the influence of the target variation on the functions of the target gene is assessed through a third treatment.

The third treatment includes: determining the degree of the influence of the target variation on the functions of the target gene by searching for the initiation codon pathogenicity reports of the target gene about the target variation.

In an embodiment, in the third treatment, if the initiation codon pathogenicity reports of the target gene with the target variation are found, the target variation information is determined as the strong loss-of-function pathogenicity evidence of the target variation. The target variation information includes the category of the target variation and the target gene with the target variation having the initiation codon pathogenicity reports.

It can be understood that in the third treatment, if the initiation codon pathogenicity reports of the target gene with the target variation are found, it can be determined that the degree of the influence of the target variation on the functions of the target gene is great, and thus the target variation information is determined as the strong loss-of-function pathogenicity evidence of the target variation.

In this case, the target variation information includes the category of target variation and the target gene with the target variation having the initiation codon pathogenicity reports, i.e., the target variation information is the category of the target variation and the consequence of the influence of the target variation on the functions of the target gene. For example, if the category of the target variation is the third category, the target variation information is that: the variation covers the first CDS but is not whole gene deletion and the target gene with the target variation has the initiation codon pathogenicity reports. Thus, the target variation information is determined as the strong loss-of-function pathogenicity evidence of the target variation (or the strength of the loss-of-function pathogenicity evidence of the target variation is: strong).

In an embodiment, in the third treatment, if the initiation codon pathogenicity reports of the target gene with the target variation are not found, the degree of the influence of the target variation on the functions of the target gene is determined based on a proportion of the region affected by the target variation.

It can be understood that in the third treatment, if the initiation codon pathogenicity reports of the target gene with the target variation are not found, the degree of the influence of the target variation on the target gene is unclear, and thus the degree of the influence of the target variation on the functions of the target gene is required to be further determined based on the proportion of the region affected by the target variation, to determine the loss-of-function pathogenicity evidence of the target variation.

In an embodiment, in the third treatment, determining the degree of the influence of the target variation on the functions of the target gene based on a proportion of the region affected by the target variation includes: when the proportion of the region affected by the target variation is greater than or equal to a preset value, determining the target variation information as the strong loss-of-function pathogenicity evidence of the target variation. The target variation information includes the category of the target variation, the target gene with the target variation having no initiation codon pathogenicity reports, and the proportion of the region affected by the target variation being greater than or equal to the preset value.

In the present embodiment, if the category of the target variation is the third category, the target variation information is that: the variation covers the first CDS but is not the whole gene deletion, the target gene with the target variation has no initiation codon pathogenicity reports, and the proportion of the region affected by the target variation is greater than or equal to the preset value. Thus, the target variation information is determined as the strong loss-of-function pathogenicity evidence of the target variation (or the strength of the loss-of-function pathogenicity evidence of the target variation is: strong).

In an embodiment, in the third treatment, the determining the degree of the influence of the target variation on the functions of the target gene based on the proportion of the region affected by the target variation further includes: when the proportion of the region affected by the target variation is smaller than the preset value, determining the target variation information as a moderate loss-of-function pathogenicity evidence of the target variation. The target variation information includes the category of the target variation, the target gene with the target variation having no initiation codon pathogenicity report, and the proportion of the region affected by the target variation being smaller than the preset value.

In the present embodiment, if the category of the target variation is the third category, the target variation information is that: the variation covers the first CDS but is not the whole gene deletion, the target gene with the target variation has no initiation codon pathogenicity report, and the proportion of the region affected by the target variation is smaller than the preset value. Thus, the target variation information is determined as the moderate loss-of-function pathogenicity evidence of the target variation (or the strength of the loss-of-function pathogenicity evidence of the target variation is: moderate).

**In** an embodiment, in the third treatment, the category of the target variation is a third category. The third category includes variations that cover the first CDS but are not the whole gene deletion.

It can be understood that in the present embodiment, when the category of the target variation is the third category, the third treatment is used for determining the degree of the influence of the target variation on the target gene.

**In** an embodiment, the degree of the influence of the target variation on the functions of the target gene is assessed through a fourth treatment.

The fourth treatment includes: determining the degree of the influence of the target variation on the functions of the target gene by predicting whether a termination codon exists in a sequence of the target gene with the target variation.

**In** an embodiment, in the fourth treatment, if it is predicted that no termination codon exists in the sequence of the target gene with the target variation, the target variation information is determined as very strong loss-of-function pathogenicity evidence. The target variation information includes the category of the target variation and non-existence of a termination codon in the sequence of the target gene with the target variation.

It can be understood that if no termination codon exists in the sequence of the target gene with the target variation, the degree of the influence of the target variation on the target gene is very great, and thus the target variation information is determined as the very strong loss-of-function pathogenicity evidence of the target variation.

**In** this case, the target variation information includes the category of the target variation and non-existence of a termination codon in the sequence of the target gene with the target variation. For example, if the category of the target variation is the fourth category, the target variation information is that: the variation covers the last CDS and is not the whole gene deletion and no termination codon exists in the sequence of the target gene with the target variation. Thus, the target variation information is determined as the very strong loss-of-function pathogenicity evidence.

In an embodiment, in the fourth treatment, if it is predicted that a termination codon exists in a sequence of the target gene with the target variation, the degree of the influence of the target variation on the functions of the target gene is assessed through a fifth treatment.

The fifth treatment includes: determining whether the target variation causes NMD.

It can be understood that if a termination codon exists in the sequence of the target gene with the target variation, the degree of the influence of the target variation on the target gene is unclear, and thus the degree of the influence of the target variation on the functions of the target gene is required to be further assessed through the fifth treatment.

In an embodiment, in the fifth treatment, if the target variation causes the NMD, the target variation information is therefore determined as a very strong loss-of-function pathogenicity evidence of the target variation. The target variation information includes the category of the target variation, existence of a termination codon in the sequence of the target gene with the target variation, and the target variation causing the NMD.

It can be understood that in the fifth treatment, if the target variation causes the NMD, the degree of the influence of the target variation on the target gene is very great, and thus the target variation information is determined as the very strong loss-of-function pathogenicity evidence of the target variation.

In the present embodiment, if the category of the target variation is the fourth category, the target variation information is that: the variation covers the last CDS and is not the whole gene deletion, a termination codon exists in the sequence of the target gene with the target variation, and the target variation causes the NMD. Thus, the target variation information is determined as the very strong loss-of-function pathogenicity evidence of the target variation.

In an embodiment, if the target variation does not cause NMD, the degree of the influence of the target variation on the functions of the target gene is assessed through a first treatment.

The first treatment includes: determining degree of the influence of the target variation on the functions of the target gene by comparing a positional relationship between the target variation and a last pathogenic variation at the 3' end of the target gene.

It can be understood that if the target variation does not cause NMD, the degree of the influence of the target variation on the target gene is unclear, and the degree of the influence of the target variation on the functions of the target gene is required to be further assessed through the first treatment.

In an embodiment, in the first treatment, if the target variation is located upstream of the last pathogenic variation at the 3' end of the target gene, then, the target variation information is determined as a strong loss-of-function pathogenicity evidence. The target variation information includes the category of the target variation, existence of a termination codon in the sequence of the target gene with the target variation, the target variation not causing NMD, and the target variation being located upstream of the last pathogenic variation at the 3' end of the target gene.

In the present embodiment, if the category of the target variation is the fourth category, the target variation information is that: the variation includes the last CDS and is not whole gene deletion, a termination codon exists in a sequence of the target gene with the target variation, the target variation does not cause NMD, and the target variation is located upstream of the last pathogenic variation at the 3' end of the target gene. Thus, the target variation information is determined as the strong loss-of-function pathogenicity evidence of the target variation.

In an embodiment, in the first treatment, if the target variation is located downstream of the last pathogenic variation at the 3' end of the target gene, the degree of the influence of the target variation on the functions of the target gene is determined based on a proportion of the region affected by the target variation.

In an embodiment, in the first treatment, determining the degree of the influence of the target variation on the functions of the target gene based on a proportion of the region affected by the target variation includes: when the proportion of the region affected by the target variation is greater than or equal to a preset value, determining the target variation information as the strong loss-of-function pathogenicity evidence of the target variation. The target variation information includes the category of the target variation, existence of a termination codon in the sequence of the target gene with the target variation, the target variation not causing the NMD, the target variation being located downstream of the last pathogenic variation at the 3' end of the target gene, and the proportion of the region affected by the target variation being greater than or equal to the preset value.

In the present embodiment, if the category of the target variation is the fourth category, the target variation information is that: the variation covers the last CDS and is not the whole gene deletion, a termination codon exists in a sequence of the target gene with the target variation, the target variation does not cause NMD, the target variation is located downstream of the last pathogenic variation at the 3' end of the target gene, and the proportion of the region affected by the target variation is greater than or equal to the preset value. Thus, the target variation information is determined as the strong loss-of-function pathogenicity evidence.

In an embodiment, the determining the degree of the influence of the target variation on the functions of the target gene based on a proportion of the region affected by the target variation further includes: when the proportion of the region affected by the target variation is smaller than the preset value, determining the target variation information as a moderate loss-of-function pathogenicity evidence of the target variation. The target variation information includes the category of the target variation, existence of a termination codon in the sequence of the target gene with the target variation, the target variation not causing the NMD, the target variation being located downstream of the last pathogenic variation at the 3' end of the target gene, and the proportion of the region affected by the target variation being smaller than the preset value

In the present embodiment, if the category of the target variation is the fourth category, the target variation information is that: the variation covers the last CDS and is not the whole gene deletion, a termination codon exists in a sequence of the target gene with the target variation, the target variation does not cause NMD, the target variation is located downstream of the last pathogenic variation at the 3' end of the target gene, and the proportion of the region affected by the target variation is smaller than the preset value. Thus, the target variation information is determined as the moderate loss-of-function pathogenicity evidence.

In an embodiment, in the fourth treatment, the category of the target variation is a fourth category.

The fourth category includes variations that cover the last CDS but are not the whole gene deletion.

It can be understood that in the present embodiment, when the category of the target variation is the fourth category, the fourth treatment is used for determining the degree of the influence of the target variation on the target gene. When the degree of the influence of the target variation on the target gene is not assessed through the fourth treatment, the fifth treatment is further used. When the degree of the influence of the target variation on the target gene cannot be assessed through the fifth treatment, the first treatment is further used for determining the degree of the influence of the target variation on the target gene.

According to a third aspect, in an embodiment, a method for correcting strength of loss-of-function pathogenicity evidence is provided. The method includes: correcting the strength of the loss-of-function pathogenicity evidence determined by any of the methods in the second aspect.

In an embodiment, the correcting includes correction based on association strength between the target gene and a disease.
In an embodiment, the correction based on the association strength between the target gene and the disease includes: if the association strength between the target gene and the disease is strong, performing no correction; if the association strength between the target gene and the disease is moderate, down-regulating the loss-of-function pathogenicity evidence by one level; and if the association strength between the target gene and the disease is weak, down-regulating the loss-of-function pathogenicity evidence by two levels.

The method of the present application determines the loss-of-function pathogenicity evidence for the target variation, and can further correct the loss-of-function pathogenicity evidence based on the association strength between the target gene and the disease, to obtain more accurate results.

According to a fourth aspect, in an embodiment, an apparatus for determining loss-of-function pathogenicity evidence is provided. The apparatus includes: a category obtaining module configured to obtain, based on an influence of a target variation on transcription, a category of the target variation; an influence determination module configured to determine, based on the category of the target variation, an influence of the target variation on functions of a target gene; and an evidence determination module configured to determine, based on a determined consequence of the influence of the target variation on the functions of the target gene, whether the target variation is the loss-of-function pathogenicity evidence.

According to a fifth aspect, in an embodiment, an apparatus for determining the strength of loss-of-function pathogenicity evidence is provided. The apparatus includes: a strength determination module configured to determine strength of the loss-of-function pathogenicity evidence obtained by the apparatus of the fourth aspect based on a degree of the influence of the target variation on the functions of the target gene.

According to a sixth aspect, in an embodiment, an apparatus for correcting the strength of loss-of-function pathogenicity evidence is provided. The apparatus includes a correcting module configured to correct the strength of the loss-of-function pathogenicity evidence determined by the apparatus of the fifth aspect.

According to a seventh aspect, in an embodiment, an apparatus for determining loss-of-function pathogenicity evidence is provided. The apparatus includes a memory configured to store programs, and a processor configured to execute the programs stored in the memory to implement any of the methods of the first aspect.

According to an eighth aspect, in an embodiment, an apparatus for determining strength of loss-of-function pathogenicity evidence is provided. The apparatus includes a memory configured to store programs, and a processor configured to execute the programs stored in the memory to implement any of the methods of the second aspect.

According to a ninth aspect, in an embodiment, an apparatus for correcting the strength of loss-of-function pathogenicity evidence is provided. The apparatus includes a memory configured to store programs, and a processor configured to execute the programs stored in the memory to implement any of the methods of the third aspect.

According to a tenth aspect, in an embodiment, a computer-readable storage medium is provided. The computer-readable storage medium has a program stored thereon. The program is executable by a processor to implement any of the methods of the first aspect, the second aspect or the third aspect.

### Example 1

In the present example, the target variation is an exon-deletion variation.

### 1. Determination of loss-of-function pathogenicity evidence

Based on the influence of the target variation on transcription, the obtained category of the target variation includes:
fifth category: the variation cannot be annotated to the coding transcript; and
sixth category: the variation can be annotated to the coding transcript, but the deletion part does not contain the coding sequence (CDS).

### 1.2 Determination of an influence of the target variation on functions of a target gene

The target variation of the fifth category or the target variation of the sixth category does not affect the functions of the target gene.

### 1.3 Determination whether the target variation information is loss-of-function pathogenicity evidence

The target variation of the fifth category or the target variation of the sixth category has no influence on the functions of the target gene. Thus, the fifth category or the sixth category is not the loss-of-function pathogenicity evidence of the exon-deletion variation.

The present example simplifies the determination of the loss-of-function pathogenicity evidence of the exon-deletion variation. Conventional methods require multiple steps to determine whether the target variation information is the loss-of-function pathogenicity evidence, for example, determining whether it causes change in the open reading frame, whether it causes the NMD, the function condition of the corresponding amino acid region, and whether it comes from the primary expression transcript. This example greatly simplifies the multi-path and multi-step determination, allowing the determination logic to be clearer.

It is understood that the embodiments of the present application can directly exclude the target variations satisfying the fifth category and the sixth category before the loss-of-function pathogenicity evidence of the target variation is determined.

### Example 2

In the present example, the target variation is an exon-deletion variation.

### 1. Determination of loss-of-function pathogenicity evidence

1.1 Based on the influence of the target variation on transcription, the obtained category of the target variation includes:
first category: the target variation results in a change in open reading frame, but does not cause NMD; and
second category: the target variation does not result in a change in open reading frame.

The method for determining whether NMD is caused is as follows: the amino acid position value 2 corresponding to the termination codon after the variation, and a theoretical critical amino acid position value 3 that causes the NMD are calculated; if the amino acid position value 2 is less than the amino acid position value 3, it is determined that the variation causes the NMD; and if the amino acid position value 2 is not less than the amino acid position value 3, it is determined that the variation does not cause the NMD. The amino acid position value 3 may derive from records in the public database (such as https://www.mutationtaster.org/). In a preferred solution, the collected amino acid position value 3 may be preprocessed to generate a custom configuration library, which can be directly called in the determination process to further improve the interpretation efficiency and reduce the waste of computing resources. It should be particularly noted that the amino acid position value **2** is predicted based on the variant CDS. Therefore, it is necessary to obtain the CDS sequence after the variation by splicing, and then determine the amino acid position value **2** based on the amino acid length of the variant CDS. In this case, the variant CDS is based on a reference CDS. The CDS deletion sequence corresponding to the variation is directly deleted according to the annotation result, and the remaining sequences are spliced in the original order to obtain the CDS sequence.

The change in the open reading frame includes that: in the CDS region, the variation sequence is a multiple of non-three nucleotides; or the variation sequence is a multiple of three nucleotides but the amino acid position value **2** is different from the theoretical termination codon position. For the exon-deletion, the theoretical termination codon position is calculated by adding 1 to the difference in the amino acid lengths of the reference sequence and the deletion sequence. That is, the open reading frame does not change only when the variation sequence is a multiple of three nucleotides and the amino acid position value **2** is the same as the theoretical termination codon position. It should be particularly noted that the situation, in which the variation sequence is a multiple of three nucleotides but the amino acid position value **2** is different from the theoretical termination codon position, is classified as the type of the change in the open reading frame, while the conventional method simply makes determination based on the result whether the variation is a multiple of three nucleotides. By determining the source of the loss-of-function pathogenicity evidence and including a special case such as deletion of a multiple of non-three nucleotides but the production of a new termination codon that leads to early termination, the loss-of-function pathogenicity evidence can be determined by further determining the degree of the influence of early stop on the gene function.

The present example achieves a more comprehensive and reasonable coverage of the loss-of-function pathogenicity evidence. Compared with the conventional interpretation method, the present example can avoid the omission of special cases and improve the accuracy of detection.

### 1.2 Determination of an influence of the target variation on functions of a target gene

When the target variation is of the first category (i.e., causing a change in the open reading frame and not causing NMD), it can be directly determined that the target variation has an influence on the functions of the target gene.

When the target variation is of the second category (i.e., not causing a change in the open reading frame), it can be directly determined that the target variation has an influence on the functions of the target gene.

### 1.3 Determination whether the target variation information is loss-of-function pathogenicity evidence

The target variation of the first category has an influence on the functions of the target gene, and the first category (causing a change in the open reading frame and not causing NMD) is determined as the loss-of-function pathogenicity evidence of the exon-deletion variation.

The target variation of the second category has an influence on the functions of the target gene, and the second category (not causing a change in the open reading frame) is determined as the loss-of-function pathogenicity evidence of the exon-deletion variation.

### 2. Determination of strength of loss-of-function pathogenicity evidence

### 2.1 Specific method for determining the degree of the influence of the target variation on the functions of the target gene

### a. Target variation of the first category: the exon-deletion variation that causes the change in the open reading frame and does not cause NMD

For the target variation of the first category, a first treatment is used to determine the degree of the influence of the target variation of the first category on the functions of the target gene: an amino acid position value 1 corresponding to an initial position of the target variation of the first category is compared with an amino acid position value 4 corresponding to the last pathogenic variation at the 3' end of the target gene. If the amino acid position value 1 is not greater than the amino acid position value 4, it is determined that the degree of the influence of the target variation of the first category on the functions of the target gene is great. It is determined that: the open reading frame being changed, NMD being not caused and the amino acid position value 1 being not greater than the amino acid position value 4 are the strong loss-of-function pathogenicity evidences of the exon-deletion variation. If the amino acid position value 1 is greater than the amino acid position value 4, the degree of the influence of the target variation of the first category on the gene function is determined to be unclear.

The amino acid position value **1** is calculated by: comparing the sequence consistency after left alignment between the reference CDS and the variant CDS; calculating the length value **3** from base A to the first inconsistent base in the reference CDS initiation codon ATG; and dividing the length value **3** by 3. If the result is an integer, the amino acid position value is 1; and if the result is not an integer, the amino acid position value **1** is the integer part plus 1. The splicing method of the variant CDS is the same as the section 1.1. The amino acid position value **4** represents the position of the amino acid corresponding to the last variation at the 3' end in the credible pathogenic variation. The credible pathogenic variation may derive from records in the public database (for example, https://www.ncbi.nlm.nih.gov/gene/), and may also derive from the experimental verification. In a preferred solution, the collected position value **4** can be preprocessed to generate a custom configuration library, which can be directly called in the determination process to further improve the interpretation efficiency and reduce the waste of computing resources.

If the degree of the influence of the target variation of the first category on the functions of the target gene is determined to be unclear, the loss-of-function pathogenicity evidence should be further determined based on a proportion of the affected region of the variation.

### b. Target variation of the second category: the exon-deletion variation that does not cause the change in the open reading frame

For the target variation of the second category, a second treatment is used to determine the influence of the target variation of the second category on the functions of the target gene:

A coverage degree between the region affected by the target variation of the second category and the important functional domain of the target gene is compared. If the region affected by the target variation overlaps with the important functional domain of the target gene, the degree of the influence of the target variation of the second category on the functions of the target gene is great. Further, it is determined that the second category and the region affected by the target variation of the second category overlapping with the important functional domain of the target gene are the strong loss-of-function pathogenicity evidences of the exon-deletion variation. If the region affected by the target variation of the second category does not overlap with the important functional domain of the target gene, the degree of the influence of the target variation of the second category on the functions of the target gene is determined to be unclear.

The initial position of the region affected by the target variation is the position value **1,** and the stop position is calculated based on: position value **1** + length value **2** - (position value **2** - 1). The calculation of the amino acid position value **1** is the same as step 2.1a, the length value **2** is the amino acid length encoded by the reference CDS, and the calculation of the amino acid position value **2** is the same as step 1.1.

The important functional domain information of the gene may derive from the records in the public database (for example, https://www.ebi.ac.uk/interpro/), and may also derive from the records in the scientific literature. In a preferred solution, the collected gene functional domain information can be preprocessed to generate a custom configuration library, which can be directly called in the determination process to further improve the interpretation efficiency and reduce the waste of computing resources.

If the influence of the target variation of the second category on the functions of the target gene is determined to be unclear, the loss-of-function pathogenicity evidence should be further determined based on a proportion of the region affected by the target variation.

In order to determine the strength of the loss-of-function pathogenicity evidence, it is a key link to determine the degree of the influence of the target variation on the functions of the target gene, especially for the variations that cause only partial deletion of the coding sequence. It cannot be directly determined whether such variations will lead to complete loss of the gene function. In the prior art, it is only generally summarized that it is required to determine whether the variation region has an influence on the gene function, which is obviously lack of operability. This step is operationally designed based on experts' consensus and guidelines, and automatically designed on this basis. It should be particularly noted that the present example fully considers the biological characteristics of the different subtypes of the exon-deletion variation and uses a differentiated determination method which is more comprehensive and reasonable. For example, the exon-deletion variation that satisfies the first category leads to the change in the open reading frame, so the functional domain information based on the reference genome is not fully applicable. That is, the degree of the influence of the target variation on the functions of the target gene may not be accurately determined even by means of the second treatment.

### 2.2 Specific method for determining the proportion of the region affected by the target variation

The proportion of the region affected by the target variation refers to the proportion of the length of the affected region of the variation to the length of the reference sequence. If the proportion is greater than or equal to a preset value (e.g., 10%), the degree of the influence of the target variation on the functions of the target gene is great. It is determined that the first category, the amino acid position value 1 being greater than the amino acid position value 4, and the proportion of the length of the affected region of the variation to the length of the reference sequence being greater than or equal to 10% (or the second category, the region affected by the target variation of the second category not overlapping with the important functional domain of the target gene, and the proportion of the length of the affected region of the variation to the length of the reference sequence being greater than or equal to 10%) are the strong loss-of-function pathogenicity evidences of the exon-deletion variation. If the proportion is smaller than the preset value (e.g., 10%), it is determined that the degree of the influence of the target variation on the functions of the target gene is small. Then, it is determined that first category, the amino acid position value 1 being greater than the amino acid position value 4, and the proportion of the length of the affected region of the variation to the length of the reference sequence being smaller than 10% (or the second category, and the region affected by the target variation of the second category not overlapping with the important functional domain of the target gene, and the proportion of the length of the affected region of the variation to the length of the reference sequence being smaller than 10%) are the moderate loss-of-function pathogenicity evidences of the exon-deletion variation. It is understood that the region affected by the target variation and the reference sequence need to be compared at the same level.

Taking the amino acid level as an example, the proportion of the amino acid length (length value 1) of the region affected by the target variation to the length of the reference amino acid sequence (length value 2) is calculated. Specifically, the length value 1 = the length value 2 - (the amino acid position value 2 - 1). The length value 2 is the length of the amino acid encoded by the reference CDS, and the calculation of the amino acid position value 2 is the same as step 1.1.

### Example 3

In the present example, the target variation is an exon-deletion variation.

### 1. Determination of loss-of-function pathogenicity evidence

### 1.1 Based on the influence of the target variation on transcription, the obtained category of the target variation further includes:

Third category: the target variation covers the first CDS and is not whole gene deletion.

The target variation of the third category belongs to a special case and can be subjected to a special treatment. There is no restriction on the sequence of the treatment of the target variation of the third category and Example 2.

### 1.2 Determination of an influence of the target variation on functions of a target gene

When the target variation is of the third category: when the first CDS is covered and it is not whole gene deletion, it can be directly determined that the target variation of the third category has an influence on the functions of the target gene.

### 1.3 Determination whether the target variation information is loss-of-function pathogenicity evidence

As the target variation of the third category has an influence on the functions of the target gene, the third category is determined as the loss-of-function pathogenicity evidence of the target variation, that is, covering the first CDS and non-whole gene deletion are the loss-of-function pathogenicity evidences of the exon-deletion variation.

### 2. Determination of strength of loss-of-function pathogenicity evidence

### 2.1 Specific determination of the degree of the influence of target variation on target gene:

For the target variation of the third category, a third treatment is used to determine the degree of the influence of the target variation of the third category on the functions of the target gene. If there are initiation codon pathogenicity reports for the target gene with the target variation of the third category, the degree of the influence of the target variation of the third category on the functions of the target gene is determined to be great. Thus, the third category and the target gene with the target variation of the third category having the initiation codon pathogenicity reports are determined as the strong loss-of-function pathogenicity evidences of the exon-deletion variation. If there are no initiation codon pathogenicity reports for the target gene with the target variation of the third category, the degree of the influence of the target variation of the third category on the functions of the target gene is determined to be unclear.

The initiation codon variation pathogenicity reports may derive from the records in the public database (for example, https://www.ncbi.nlm.nih.gov/gene/), and may also derive from the literature report. In a preferred solution, the collected pathogenic variation information of the initiation codon can be preprocessed to generate a custom configuration library, which can be directly called in the determination process to further improve the interpretation efficiency and reduce the waste of computing resources.

If the influence of the target variation on the functions of the target gene is determined to be unclear, the proportion of the region affected by the target variation needs to be further determined to determine the loss-of-function pathogenicity evidence.

### 2.2 Specific method for determining the proportion of the region affected by the target variation

The proportion of the region affected by the target variation refers to the proportion of the length of the region affected by the target variation to the length of the reference sequence. If the proportion is greater than or equal to a preset value (e.g., 10%), the degree of the influence of the target variation on the functions of the target gene is great. Thus, it is determined that the third category, the target gene with the target variation of the third category having no initiation codon pathogenicity reports, and the proportion of the length of the affected region of the variation to the length of the reference sequence being greater than or equal to 10% are determined as the strong loss-of-function pathogenicity evidences of the exon-deletion variation. If the proportion is smaller than the preset value (e.g., 10%), the degree of the influence of the target variation of the third category on the functions of the target gene is small. Thus, it is determined that the third category, the target gene with the target variation of the third category having no initiation codon pathogenicity reports, and the proportion of the length of the affected region of the variation to the length of the reference sequence being smaller than10% are determined as the moderate loss-of-function pathogenicity evidences of the exon-deletion variation. It is understood that the region affected by the target variation and the reference sequence need to be compared at the same level.

Taking the amino acid level as an example, the proportion of the amino acid length (length value 1) of the affected region of the variation to the length (length value 2) of the reference amino acid sequence is calculated. The length value 1 is calculated by dividing the length of the nucleotide sequence of the deletion CDS compared with the reference CDS by 3. If the value is an integer, the length value is 1; and if the value is not an integer, the length value 1 is the integer part plus 1.

It should be particularly noted that, compared with section 2.2 in example 2, the region affected by the target variation is calculated in a different way. According to the subdivided variation types and the unique biological function effects of each type, the present example defines and refines the affected region, and carries out differentiated design, which is more scientific and reasonable.

Exon-deletion variations that cover the first CDS and are not whole gene deletion usually involve multiple functional elements and have a variety of influences on the gene function, which may involve the change in the initiation codon and fragment deletion. The present example creatively designs a hierarchical determination of the exon-deletion variations that include the first CDS, and fully covers various cases. The present example fully mines and utilizes the public data, quickly determines the pathogenicity evidence of part of the variations through the pathogenicity determination of the initiation codon, and quickly determines the strength of the pathogenicity evidence. For those that cannot be determined quickly, the present example determines the evidence and the strength of the evidence from the perspective of whether it causes the loss of gene function fundamentally by further creatively combining the proportion of region affected by the target variation.

### Example 4

In the present example, the target variation is an exon-deletion variation.

### 1. Determination of loss-of-function pathogenicity evidence

### 1.1 Based on the influence of the target variation on transcription, the obtained category of the target variation further includes:

Fourth category: the target variation covers the last CDS and is not whole gene deletion.

The exon-deletion variation of the fourth category belongs to a special case and can be subjected to a special treatment. There is no restriction on the sequence of the treatment of the exon-deletion variation of the fourth category, Example 2 and embodiment 3.

### 1.2 Determination of an influence of the target variation on functions of a target gene

When the exon-deletion variation is of the fourth category: when the last CDS is covered and it is not whole gene deletion, it can be directly determined that the exon-deletion variation of the fourth category has an influence on the functions of the target gene.

### 1.3 Determination whether the target variation information is loss-of-function pathogenicity evidence

If the target variation of the fourth category has an influence on the functions of the target gene, it is determined that the fourth category is the loss-of-function pathogenicity evidence of the exon-deletion variation. That is, covering the last CDS and non-whole gene deletion are the loss-of-function pathogenicity evidences of the exon-deletion variation.

### 2. Determination of strength of loss-of-function pathogenicity evidence

### 2.1 Specific method for determining the degree of the influence of the target variation on the functions of the target gene

For the target variation of the fourth category, a fourth treatment is used to determine the degree of the influence of the target variation of the fourth category on the functions of the target gene:

If it is predicted that no termination codon exists in the sequence of the target gene with the target variation of the fourth category, it is determined that the degree of the influence of the target variation of the fourth category on the functions of the target gene is very great. Then, it is determined that the fourth category and non-existence of a termination codon in the sequence of the target gene that is predicted to have the target variation of the fourth category are the very strong evidences of pathogenicity of the exon-deletion variation. If it is predicted that a termination codon exists in a sequence of the target gene with the target variation of the fourth category, the degree of the influence of the target variation of the fourth category on the functions of the target gene is determined to be unclear.

In the conventional annotation process, the specific cases of the termination codon are not considered. However, the absence of the termination codon triggers Non-Stop Decay (NSD), which further affect the gene functions. The present example comprehensively considers the special type that a termination codon is absent after the variation, and identifies the loss-of-function pathogenicity evidence from the source of the influence of the variation on the gene function. Such a particular type of variation corresponds to the very strong loss-of-function pathogenicity evidence, and according to the experts' consensus and guidelines, only a small amount of other evidence is needed to determine the association of this variation with diseases. It can be seen that, compared with the conventional interpretation method, the present example can avoid omissions and improve the accuracy of detection.

2.2 If the degree of the influence of the exon-deletion variation of the fourth category on the functions of the target gene is unclear, the degree of the influence of the target variation of the fourth category on the functions of the target gene is further assessed through the fifth treatment. The fifth treatment includes: determining whether the target variation of the fourth category causes the NMD.

If the target variation of the fourth category causes the NMD, the degree of the influence of the target variation of the fourth category on the target variation function is very great. Thus, it is determined that the fourth category, existence of a termination codon in the sequence of the target gene that is predicted to have the target variation of the fourth category, and the target variation of the fourth category causing the NMD are the very strong loss-of-function pathogenicity evidences of the exon-deletion variation.

If the target variation of the fourth category does not cause the NMD, the first treatment is performed on the target variation of the fourth category to determine the influence of the target variation of the fourth category on the functions of the target gene. The above determination method adopts the determination method of the first treatment on the target variation of the first category as described in section 2.1 of Example 2.

The amino acid position value 1 corresponding to the initial position of the target variation of the fourth category is compared with the amino acid position value 4 corresponding to the last pathogenic variation at the 3' end of the target gene. If the amino acid position value 1 is not greater than the amino acid position value 4, it is determined that the degree of the influence of the target variation of the fourth category on the functions of the target gene is great. It is determined that the fourth category, existence of a termination codon exists in a sequence of the target gene that is predicted to have the target variation of the fourth category, the target variation of the fourth category not causing the NMD, and the amino acid position value 1 being not greater than the amino acid position value 4 are the strong loss-of-function pathogenicity evidences of the exon-deletion variation. If the amino acid position value 1 is greater than the amino acid position value 4, the degree of the influence of the target variation of the first category on the gene function is determined to be unclear.

2.3 The determination method of the proportion of the region affected by the target variation of the fourth category is the same as section 2.2 in Example 2, which will not be repeated here.

The proportion of the region affected by the target variation of the fourth category refers to the proportion of the length of the affected region of the variation to the length of the reference sequence. If the proportion is greater than or equal to a preset value (e.g., 10%), the degree of the influence of the target variation of the fourth category on the functions of the target gene is great. It is determined that the fourth category, existence of a termination codon exists in a sequence of the target gene that is predicted to have the target variation of the fourth category, the target variation of the fourth category not causing the NMD, the amino acid position value 1 being not greater than the amino acid position value 4, and the proportion of the length of the affected region of the variation to the length of the reference sequence being greater than or equal to 10% are the strong loss-of-function pathogenicity evidences of the exon-deletion variation. If the proportion is smaller than a preset value (e.g., 10%), the degree of the influence of the target variation of the fourth category on the functions of the target gene is determined to be small. It is determined that the fourth category, existence of a termination codon exists in a sequence of the target gene that is predicted to have the target variation of the fourth category, the target variation of the fourth category not causing the NMD, the amino acid position value 1 being not greater than the amino acid position value 4, and the proportion of the length of the affected region of the variation to the length of the reference sequence being smaller than 10% are the moderate loss-of-function pathogenicity evidences of the exon-deletion variation.

### Example 5

In the present example, the target variation is an exon-deletion variation.

### 1. Determination of loss-of-function pathogenicity evidence

### 1.1 According to the influence of the target variation on transcription, the obtained category of the target variation also includes:

seventh category: an exon-deletion variation that causes the deletion of all coding sequences.

### 1.2 Determination of an influence of the target variation on functions of a target gene

For the target variation of the seventh category: the exon-deletion variation causes the deletion of all coding sequences, and thus it can be directly determined that the target variation of the seventh category has an influence on the functions of the target gene.

### 1.3 Determination whether the target variation information is loss-of-function pathogenicity evidence

The target variation of the seventh category has an influence on the functions of the target gene, and the seventh category can be directly determined as the loss-of-function pathogenicity evidence of the target variation.

### 2. Determination of strength of loss-of-function pathogenicity evidence

When the target variation is of the seventh category, the seventh category can be directly determined as the very strong loss-of-function pathogenicity evidence of the target variation.

In the present example, for the target variation of the seventh category, it can be directly determined that the target variation of the seventh category has an influence on the functions of the target gene. In this regard, the seventh category can be directly determined as the very strong loss-of-function pathogenicity evidence of the target variation.

The conventional method conducts determination by whether the deletion of all genes is caused. However, a complete gene includes a coding region and a non-coding region. For genes with loss-of-function pathogenic mechanisms, the deletion of the coding sequence alone can indicate that these genes are unable to synthesize biologically functional transcripts, which further leads to the loss of function of the corresponding proteins. The present example can more fully include the exon-deletion variations satisfying the very strong loss-of-function pathogenicity evidence.

### Example 6

### 1. Determination of loss-of-function pathogenicity evidence

In the present example, the target variation is an exon-deletion variation.

### 1.1 According to the influence of the target variation on transcription, the obtained category of the target variation also includes:

eighth category: the target variation causes a change in the open reading frame and causes the NMD.

### 1.2 Determination of an influence of the target variation on functions of a target gene

For the target variation of the eighth category, it can be directly determined that the target variation of the eighth category has an influence on the functions of the target gene.

### 1.3 Determination whether the target variation information is loss-of-function pathogenicity evidence

The target variation of the eighth category has an influence on the functions of the target gene, and the target variation of the eighth category can be directly determined as the loss-of-function pathogenicity evidence.

### 2. Determination of strength of loss-of-function pathogenicity evidence

When the target variation is of the eighth category, the eighth category can be directly determined as the very strong loss-of-function pathogenicity evidence of the target variation.

In the present example, for the target variation of the eighth category, it can be directly determined that the target variation of the eighth category has an influence on the functions of the target gene, and thus, the eighth category can be directly determined as the very strong loss-of-function pathogenicity evidence of the target variation.

The determination principle of whether the open reading frame is changed is the same as section 2.1 in Example 2.

In the embodiments of the present application, the classification order of the target variations of the first category to the eighth category and the corresponding order of the method for determining the influence of the target variation on the functions of the target gene are not specifically limited.

The loss-of-function pathogenicity evidence is corrected based on the association strength between the target gene and the disease. The association strength between the gene and the disease may derive from the records in the public database (such as https://www.clinicalgenome.org/, and https://www.omim.org/), and may also derive from the literature report. A specific correction method includes: when the association strength between the target gene and the disease is strong, performing no correction; when the association strength between the target gene and the disease is moderate, down-regulating the loss-of-function pathogenicity evidence by one level (for example, the very strong loss-of-function pathogenicity evidence is corrected as the strong loss-of-function pathogenicity evidence, and the strong loss-of-function pathogenicity evidence is corrected as the moderate loss-of-function pathogenicity evidence, and so on); and when the association strength between the target gene and the disease is weak, down-regulating the loss-of-function pathogenicity evidence by two levels (for example, the very strong loss-of-function pathogenicity evidence is corrected as the moderate loss-of-function pathogenicity evidence, and the strong loss-of-function pathogenicity evidence is corrected as the supportive loss-of-function pathogenicity evidence, and so on).

The beneficial effects obtained by the embodiments of the present application include the follows.

By clarifying the application situation of the evidence and the strength of the evidence, the standardization and accuracy of the use of the evidence can not only be improved, and the investment in manual inquiry of other types of evidence can also be greatly reduced, improving the efficiency and consistency of interpretation, and further improving mass and automatic interpretation.

It is understood by those skilled in the art that all or part of the functions of the methods in the above embodiments may be implemented by hardware or by computer programs. When all or part of the functions in the above embodiments are realized by the computer programs, the programs may be stored in a computer-readable storage medium which may include: a read-only memory, a random access memory, a disk, an optical disc, a hard disk, etc., and the programs are executed by a computer to achieve the above functions. For example, when the programs are stored in a memory of a device, all or part of the above functions can be implemented when the programs in the memory are executed by a processor. In addition, when all or part of the functions in the above embodiments are achieved by the computer programs, the programs may also be stored in a storage medium such as a server, another computer, a disk, an optical disc, a flash drive or a portable hard drive, and downloaded or copied and saved to the memory of a local device, or the system of the local device is updated. When the programs in the memory are executed by a processor, all or part of the functions in the above embodiments can be achieved.

The present application is explained by means of the specific examples above, which are only intend to help to understand the present application, rather than limiting the present application. Those skilled in the art in the present application, based on the concept of the present application, can make several simple deductions, variations or replacements.

## Claims

1. A method for determining loss-of-function pathogenicity evidence, the method comprising:
Step S101 of obtaining, based on an influence of a target variation on transcription, a category of the target variation;
Step S102 of determining, based on the category of the target variation, an influence of the target variation on functions of a target gene, wherein the target gene is a gene where the target variation occurs; and
Step S103 of determining, based on a consequence of the influence obtained in the Step S102 of the target variation on the functions of the target gene, whether target variation information is loss-of-function pathogenicity evidence of the target variation,
wherein the target variation information is the category of the target variation and/or the consequence of the influence in the target variation information.

2. The method according to claim 1, wherein the target variation is a single- or multiple-exon deletion variation.

3. The method according to claim 1 or 2, wherein the influence of the target variation on transcription comprises:
whether the target variation causes a change in an Open Reading Frame (ORF); and/or
whether the target variation causes Nonsense-Mediated mRNA Decay (NMD).

4. The method according to any one of claims 1 to 3, wherein the category of the target variation comprises one or more of a first category, a second category, a third category, and a fourth category, wherein:
the first category comprises variations that cause a change in ORF and do not cause NMD;
the second category comprises variations that do not cause a change in ORF;
the third category comprises variations that cover a first coding sequence (CDS) but are not a whole gene deletion; and
the fourth category comprises variations that cover a last CDS but are not the whole gene deletion.

5. The method according to any one of claims 1 to 4, wherein:
if the target variation has an influence on the functions of the target gene, the target variation information is determined as the loss-of-function pathogenicity evidence of the target variation; and
if the target variation has no influence on the functions of the target gene, the target variation information is determined to be not the loss-of-function pathogenicity evidence of the target variation.

6. The method according to claim 5, wherein when the category of the target variation comprises one or more of a first category, a second category, a third category, and a fourth category, the target variation is determined to have an influence on the functions of the target gene, and the category of the target variation is determined to be the loss-of-function pathogenicity evidence of the target variation;
the first category comprises variations that cause a change in ORF and do not cause the NMD;
the second category comprises variations that do not cause a change in ORF;
the third category comprises variations that cover a first CDS but are not a whole gene deletion; and
the fourth category comprises variations that cover a last CDS but are not the whole gene deletion.

7. The method according to claim 5, wherein when the category of the target variation comprises a fifth category and/or a sixth category, the target variation has no influence on the functions of the target gene, and the category of the target variation is not the loss-of-function pathogenicity evidence of the target variation;
the fifth category comprises variations that are uncapable of being annotated to coding transcripts; and
the sixth category comprises variations that are capable of being annotated to the coding transcripts and enable a region affected by the target variation to not comprise a coding sequence.

8. A method for determining strength of loss-of-function pathogenicity evidence, the comprising:
Step S104 of determining strength of the loss-of-function pathogenicity evidence obtained by the method according to any one of claims 1 to 7 based on a degree of the influence of the target variation on the functions of the target gene.

9. The method according to claim 8, wherein the degree of the influence of the target variation on the functions of the target gene is assessed through a first treatment or a second treatment, wherein:
the first treatment comprises: determining the degree of the influence of the target variation on the functions of the target gene by comparing a positional relationship between the target variation and a last pathogenic variation at the 3' end of the target gene; and
the second treatment comprises: determining the degree of the influence of the target variation on the functions of the target gene by comparing a positional relationship between a region affected by the target variation and an important functional domain of the target gene.

10. The method according to claim 9, wherein:
in the first treatment, if the target variation is located upstream of the last pathogenic variation at the 3' end of the target gene, the target variation information is determined as a strong loss-of-function pathogenicity evidence of the target variation, the target variation information comprising the category of the target variation and the target variation being located upstream of the last pathogenic variation at the 3' end of the target gene; and
in the second treatment, if the region affected by the target variation overlaps with the important functional domain of the target gene, the target variation information is determined as a strong loss-of-function pathogenicity evidence of the target variation, the target variation information comprising the category of the target variation and the region affected by the target variation overlapping with the important functional domain of the target gene.

11. The method according to claim 9 or 10, wherein:
in the first treatment, if the target variation is located downstream of the last pathogenic variation at the 3' end of the target gene, the degree of the influence of the target variation on the functions of the target gene is determined based on a proportion of the region affected by the target variation;
in the second treatment, if the region affected by the target variation does not overlap with the important functional domain of the target gene, the degree of the influence of the target variation on the functions of the target gene is determined based on a proportion of the region affected by the target variation;
preferably, determining the degree of the influence of the target variation on the functions of the target gene based on the proportion of the region affected by the target variation comprises: when the proportion of the region affected by the target variation is greater than or equal to a preset value, determining the target variation information as the strong loss-of-function pathogenicity evidence of the target variation, wherein:
in the first treatment, the target variation information comprises the category of the target variation, the target variation being located downstream of the last pathogenic variation at the 3' end of the target gene, and the proportion of the region affected by the target variation being greater than or equal to the preset value; and
in the second treatment, the target variation information comprises the category of the target variation, the region affected by the target variation not overlapping with the important functional domain of the target gene, and the proportion of the region affected by the target variation being greater than or equal to the preset value;
preferably, said determining the degree of the influence of the target variation on the functions of the target gene based on the proportion of the region affected by the target variation further comprises: when the proportion of the region affected by the target variation is smaller than the preset value, determining the target variation information as a moderate loss-of-function pathogenicity evidence of the target variation, wherein:
in the first treatment, the target variation information comprises the category of the target variation, the target variation being located downstream of the last pathogenic variation at the 3' end of the target gene, and the proportion of the region affected by the target variation being smaller than the preset value;
in the second treatment, the target variation information comprises the category of the target variation, the region affected by the target variation not overlapping with the important functional domain of the target gene, and the proportion of the region affected by the target variation being smaller than the preset value.

12. The method according to any one of claims 9 to 11, wherein:
in the first treatment, the category of the target variation is a first category, the first category comprising variations that cause a change in ORF and do not cause the NMD; and
in the second treatment, the category of the target variation is a second category, the second category comprising variations that do not cause a change in ORF.

13. The method according to claim 8, wherein the degree of the influence of the target variation on the functions of the target gene is assessed through a third treatment, wherein:
the third treatment comprises: determining the degree of the influence of the target variation on the functions of the target gene by searching for initiation codon pathogenicity reports of the target gene with the target variation.

14. The method according to claim 13, wherein in the third treatment, if the initiation codon pathogenicity reports of the target gene with the target variation are found, the target variation information is determined as the strong loss-of-function pathogenicity evidence of the target variation, the target variation information comprising the category of the target variation and the target gene with the target variation having the initiation codon pathogenicity reports.

15. The method according to claim 13 or 14, wherein in the third treatment, if the initiation codon pathogenicity reports of the target gene with the target variation are not found, the degree of the influence of the target variation on the functions of the target gene is determined based on a proportion of the region affected by the target variation;
preferably, determining the degree of the influence of the target variation on the functions of the target gene based on the proportion of the region affected by the target variation comprises: when the proportion of the region affected by the target variation is greater than or equal to a preset value, determining the target variation information as the strong loss-of-function pathogenicity evidence of the target variation, the target variation information comprising the category of the target variation, the target gene with the target variation having no initiation codon pathogenicity reports, and the proportion of the region affected by the target variation being greater than or equal to the preset value; and
preferably, said determining the degree of the influence of the target variation on the functions of the target gene based on a proportion of the region affected by the target variation further comprises: when the proportion of the region affected by the target variation is smaller than the preset value, determining the target variation information as a moderate loss-of-function pathogenicity evidence of the target variation, the target variation information comprising the category of the target variation, the target gene with the target variation having no initiation codon pathogenicity report, and the proportion of the region affected by the target variation being smaller than the preset value.

16. The method according to any one of claims 13 to 15, wherein the category of the target variation is a third category, the third category comprising variations that cover a first CDS but are not a whole gene deletion.

17. The method according to claim 8, wherein the degree of the influence of the target variation on the functions of the target gene is assessed through a fourth treatment, wherein:
the fourth treatment comprising: determining the degree of the influence of the target variation on the functions of the target gene by predicting whether a termination codon exists in a sequence of the target gene with the target variation.

18. The method according to claim 17, wherein in the fourth treatment, if no termination codon exists in the sequence of the target gene with the target variation, the target variation information is determined as a very strong loss-of-function pathogenicity evidence, the target variation information comprising the category of the target variation and non-existence of a termination codon in the sequence of the target gene with the target variation.

19. The method according to claim 17 or 18, wherein if a termination codon exists in a sequence of the target gene with the target variation, the degree of the influence of the target variation on the functions of the target gene is assessed through a fifth treatment, wherein:
the fifth treatment comprises: determining whether the target variation causes NMD.

20. The method according to claim 19, wherein in the fifth treatment, if the target variation causes the NMD, the target variation information is determined as a very strong loss-of-function pathogenicity evidence of the target variation, the target variation information comprising the category of the target variation, existence of a termination codon in the sequence of the target gene with the target variation, and the target variation causing the NMD.

21. The method according to claim 19 or 20, wherein if the target variation does not cause the NMD, the degree of the influence of the target variation on the functions of the target gene is assessed through a first treatment, wherein:
the first treatment comprises: determining the degree of the influence of the target variation on the functions of the target gene by comparing a positional relationship between the target variation and a last pathogenic variation at the 3' end of the target gene;
preferably, in the first treatment, if the target variation is located upstream of the last pathogenic variation at the 3' end of the target gene, then, the target variation information is determined as a strong loss-of-function pathogenicity evidence, the target variation information comprising the category of the target variation, existence of a termination codon exists in the sequence of the target gene with the target variation, the target variation not causing the NMD, and the target variation being located upstream of the last pathogenic variation at the 3' end of the target gene;
preferably, in the first treatment, if the target variation is located downstream of the last pathogenic variation at the 3' end of the target gene, the degree of the influence of the target variation on the functions of the target gene is determined based on a proportion of the region affected by the target variation;
preferably, determining the degree of the influence of the target variation on the functions of the target gene based on the proportion of the region affected by the target variation comprises: when the proportion of the region affected by the target variation is greater than or equal to a preset value, determining the target variation information as the strong loss-of-function pathogenicity evidence of the target variation, the target variation information comprising the category of the target variation, existence of a termination codon in the sequence of the target gene with the target variation, the target variation not causing the NMD, the target variation being located downstream of the last pathogenic variation at the 3' end of the target gene, and the proportion of the region affected by the target variation being greater than or equal to the preset value; and
preferably, said determining the degree of the influence of the target variation on the functions of the target gene based on a proportion of the region affected by the target variation further comprises: when the proportion of the region affected by the target variation is smaller than the preset value, determining the target variation information as a moderate loss-of-function pathogenicity evidence of the target variation, the target variation information comprising the category of the target variation, existence of a termination codon in the sequence of the target gene with the target variation, the target variation not causing the NMD, the target variation being located downstream of the last pathogenic variation at the 3' end of the target gene, and the proportion of the region affected by the target variation being smaller than the preset value.

22. The method according to any one of claims 17 to 21, wherein the category of the target variation is a fourth category, the fourth category comprising variations that cover a last CDS but are not the whole gene deletion.

23. A method for correcting strength of loss-of-function pathogenicity evidence, the method comprising:
Step S105 of correcting strength of the loss-of-function pathogenicity evidence determined by the method according to any one of claims 8 to 22.

24. The method according to claim 23, wherein said correcting comprises correction based on association strength between the target gene and a disease.

25. The method according to claim 22, wherein the correction based on the association strength between the target gene and the disease comprises:
if the association strength between the target gene and the disease is strong, performing no correction;
if the association strength between the target gene and the disease is moderate, down-regulating the loss-of-function pathogenicity evidence by one level; and
if the association strength between the target gene and the disease is weak, down-regulating the loss-of-function pathogenicity evidence by two levels.

26. An apparatus for determining loss-of-function pathogenicity evidence, comprising:
a category obtaining module configured to obtain, based on an influence of a target variation on transcription, a category of the target variation;
an influence determination module configured to determine, based on the category of the target variation, an influence of the target variation on functions of a target gene; and
an evidence determination module configured to determine, based on a determined consequence of the influence of the target variation on the functions of the target gene, whether the target variation is the loss-of-function pathogenicity evidence.

27. An apparatus for determining strength of loss-of-function pathogenicity evidence, comprising:
a strength determination module configured to determine strength of the loss-of-function pathogenicity evidence obtained by the apparatus according to claim 26 based on a degree of the influence of the target variation on the functions of the target gene.

28. An apparatus for correcting loss-of-function pathogenicity evidence, comprising:
a correcting module configured to correct the strength of the loss-of-function pathogenicity evidence determined by the apparatus according to claim 27.

29. An apparatus for determining loss-of-function pathogenicity evidence, comprising:
a memory configured to store programs; and
a processor configured to execute the programs stored in the memory to implement the method according to claims 1 to 7.

30. An apparatus for determining strength of loss-of-function pathogenicity evidence, comprising:
a memory configured to store programs; and
a processor configured to execute the programs stored in the memory to implement the method according to claims 8 to 22.

31. An apparatus for correcting strength of loss-of-function pathogenicity evidence, comprising:
a memory configured to store programs; and
a processor configured to execute the programs stored in the memory to implement the method according to claims 23 to 25.

32. A computer-readable storage medium, having a program stored thereon, the program is executable by a processor to implement the method according to any one of claims 1 to 25.
